# EUROPEAN PATENT APPLICATION

(11) **EP 3 636 235 A1**
(43) Date of publication of application: **15.04.2020**
(21) Application number: 18814285.5
(22) Date of filing: 16.05.2018
(51) Int. Cl.: A61F 13/44, A61F 13/42, A61B 5/00, G08B 21/20, G06Q 50/22, A61F 13/84

(54) **ABSORPTION PRODUCT PROVIDED WITH TERMINAL UNIT**

(30) Priority: 05.06.2017 KR 20170069600; 28.02.2018 KR 20180024454
(71) Applicant: Craders Co.,Ltd, Seoul 02841 (KR)
(72) Inventor: LEE, Ui Cheol, Seoul 06241 (KR)
(74) Representative: Betten & Resch
(86) International application number: PCT/KR2018/005600
(87) International publication number: WO 2018/225960

(57) **Abstract**

An absorption product of the present invention includes: a sheet part having two or more patterns that are formed to be spaced apart from each other in an excretion area, which holds excretion of a user, and conduct electricity; and a terminal unit having three or more terminals physically being in contact with the patterns and being attached/detached to/from the sheet part, in which the terminals and the patterns may be formed such that at least one terminal physically comes in contact with each of the two or more patterns spaced apart from each other when the terminal unit is mounted on the sheet part, and a sensing module generating a sensing signal sensing the excretion and providing the sensing signal to the terminals being in contact with the patterns is provided in the terminal unit.

## Description

### Technical Field

The present invention relates to an absorption product that holds excretion discharged from the user's body.

### Background Art

A diaper is an absorptive product that the elderly or people with disability wear and there are a disposable diaper, training pants, pants for urinary incontinence, etc. These absorptive products are all used separately in accordance with the lifestyle or the nursing level of the wearers.

For example, a disposable diaper holds and absorbs excretion without leaking the excretion for people who has difficulty in excreting by themselves such as the elderly or people with disability, so such a disposable diaper is used as an assistant product for excretion etc., or a sanitary product for nursing care.

Urine may be an important datum in finding out the health condition of a user. Accordingly, a method of sensing and managing urine of a user is required.

### Disclosure

### Technical Problem

The present invention provides an absorption product that senses excretion of a user.

### Technical Solution

An absorption product of the present invention includes: a sheet part having two or more patterns that are formed to be spaced apart from each other in an excretion area, which holds excretion of a user, and conduct electricity; and a terminal unit having three or more terminals physically being in contact with the patterns and being attached/detached to/from the sheet part, in which the terminals and the patterns may be formed such that at least one terminal physically comes in contact with each of the two or more patterns spaced apart from each other when the terminal unit is mounted on the sheet part, and a sensing module generating a sensing signal sensing the excretion and providing the sensing signal to the terminals being in contact with the patterns is provided in the terminal unit.

### Advantageous Effects

The absorption product of the present invention may include a sheet part and a terminal unit being attached/detached to/from the sheet part.

The terminal unit can sense excretion of a user using a pattern formed on the sheet part.

The terminal unit can determine whether there is a problem with the sheet part using a plurality of terminals being in contact with one pattern. An absorption product that can accurately sense excretion on the basis of whether there is a problem with the sheet art, and can effectively prevent a safety accident such as an electric shock can be provided.

Further, the terminal unit can determine an alignment state between the sheet part and the terminal unit using a plurality of terminals being in contact with one pattern. The terminal unit can induce the terminal unit to be mounted right on the sheet part by informing a user of the determined alignment state.

The terminal unit can transmit visual information sensing excretion and recognition information of the terminal unit to a server.

A user wearing the sheet part can be specified using the recognition information of the terminal unit. The excretion state of the specified user can be remotely controlled.

The terminal can find out the number of replaced sheet part and can transmit information about the number of the replaced sheet part and visual information with the sheet part replaced to a server. The information about the number of the replaced sheet parts can be used to establish policies such as determining an allowance such as medical insurance.

### Description of Drawings

FIG. 1 is a schematic view showing an absorption product of the present invention.
FIG. 2 illustrates schematic views showing a cross-section of a sheet part.
FIG. 3 is a block diagram showing a terminal unit.
FIG. 4 is a schematic view showing a first terminal and a second terminal being contact with a specific pattern.
FIG. 5 illustrates schematic views showing an alignment state between patterns and terminals.
FIGS. 6 and 7 are schematic views showing a terminal unit of the present invention.
FIG. 8 is a schematic view showing another terminal unit of the present invention.
FIG. 9 illustrates schematic views showing a state in which a sheet part is disposed between elastic parts and terminals.
FIG. 10 is a schematic view showing a comparative embodiment of the present invention.

### Mode for Invention

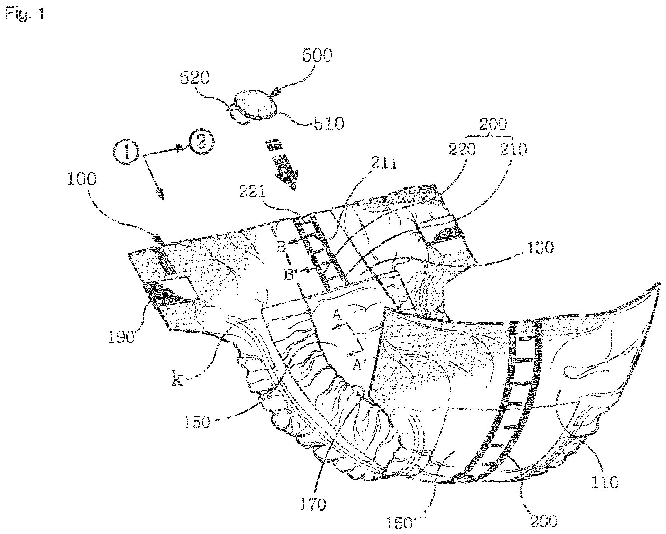

FIG. 1 is a schematic view showing an absorption product of the present invention.

The absorption product shown in FIG. 1 may include a sheet part 100 and a terminal unit 500.

The sheet part 100 may have a pattern 200 through which electricity is conducted. Two or more patterns 200 may be formed to be spaced apart from each other in an excretion area k of the sheet part 100 that holds excretion.

A user may include a person or an animal that wears the sheet part 100.

The excretion of a user may include excrement, urine, sweat, menstrual excretion, tears, saliva, etc. In order to hold the excretions, the sheet part 100 may include a diaper or a pad that holds excrement and urine, a pad or a patch that is attached to a specific position of the user's body and holds sweat, a hygienic band that holds menstrual excretion, an eye patch that holds tears, a mask that holds saliva, etc.

The sheet part 110 shown in the drawings may include a diaper that is put between the legs of a user to hold excretion and urine. The parts shown in the figures may be applied in the same way to other kinds of sheet part 100 such as a hygienic band.

A pair of tape fasteners 190 may be disposed at an edge of the diaper. The tape of the taper fasteners 190 can adhere to the other edge of the diaper.

A pair of cuffs 170 being erect upward to prevent leakage may be disposed at both sides of the center portion of the diaper.

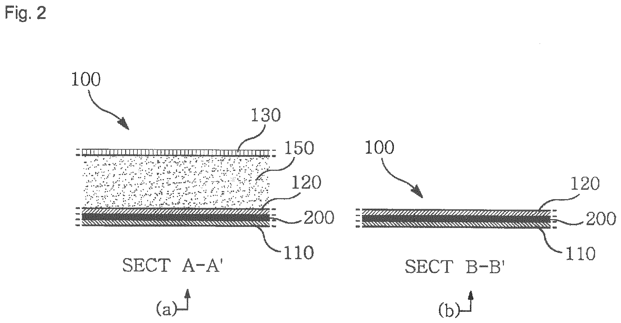

FIG. 2 illustrates schematic views showing a cross-section of the sheet part 100.

The sheet part 100 may include a first sheet part 100 made of a material at least partially not passing excretion and a second sheet 120 stacked on a side of the first sheet 110 that is supposed to face a user and made of a material passing excretion.

For example, the first sheet 110 may include a material such as vinyl and film that do not leak excretion of a user, for example, polyethylene.

The first sheet 110 may be partially changed in shape in consideration of the wearing position of the sheet part 100. The first sheet 110 may be formed fundamentally in a plate shape wider than the excretion organ in order to securely hold a large amount of excretion without leakage.

An absorber 150 may be added to increase the ability to absorbing excretion.

The absorber 150 may be stacked on a side of the second sheet 120 that is supposed to face a user. The absorber 150 may include materials such as cotton, SAP (Super Absorbent Polymer), a tissue, etc.

The absorber 150 made of cotton, etc. easily slides on the first sheet 110 made of vinyl. In this case, the cotton particles, SAP particles, and tissue particles constituting the absorber 150 are changed in position without being fixed with respect to the first sheet 110 due to the sliding, so an abnormal phenomenon that the absorber 150 is biased to any one side with the other side empty in the excretion area may occur.

The second sheet 120 that is easily bonded to the first sheet 110 made of vinyl may be used to prevent a position change of the particles of the absorber 150. For example, the second sheet 120 that is brought in contact with cotton may include fabric passing excretion and limiting a position change of the cotton.

When the absorber 150 is provided, a third sheet 130 may be added to prevent a phenomenon that the absorber 150 is separated from the second sheet 120.

The third sheet 130 is stacked on a side of the absorber 150 that is supposed to face a user, and may include a material that passes excretion. The center portion of the third sheet 130 covers the absorber 150 and the edges of the third sheet 130 can be bonded directly to the first sheet 110 or the second sheet 120.

The center portion of the sheet part 100 including the absorber 150, as shown in FIG. 2(a) showing the cross-section of line A-A' of FIG. 1, may formed in a structure in which the first sheet 110, the a pattern 200, the second sheet 120, the absorber 150, and the third sheet 130 are sequentially stacked.

The edges of the sheet part 100, as shown in FIG. 2(b) showing the cross-section of line B-B' of FIG. 1, may be formed in a structure in which the first sheet 110, the pattern 200, and the second sheet 120 are sequentially stacked. The third sheet 130 may be additionally stacked on the second sheet 120 at the edges of the sheet part 100.

The third sheet 130 can function as a cover that prevents separation of the absorber 150 from the first sheet 110 or the second sheet 120. Further, the third sheet 130 can prevent a phenomenon that the cotton particles, the SAP particles, and the tissue particles constituting the absorber 140 stick to the user's body or excretion absorbed in the absorber 150 sticks back to the user's body.

The absorber 150 and the pattern 200 may be formed in the excretion area. The first sheet 110, the second sheet 120, the absorber 150, and the third sheet 130 may be formed to have areas wider than the excretion area. However, the absorber 150 may be formed to fit to the size of the excretion area to reduce the cost. In other words, the absorber 150 may be formed to have an area smaller than the first sheet 110, the second sheet 120, and the third sheet 130. If the area of the absorber 150 is small, the edges of the third sheet 130 can be easily bonded to the edges of the second sheet 120.

According to this configuration described above, the sheet part 100 in which the first sheet 110, the second sheet 120, the absorber 150, and the third sheet 130 are sequentially stacked in accordance with the direction facing the user's body from the outside can be provided.

Since the second sheet 120, the absorber 150, and the third sheet 130 include the material that passes excretion, excretion discharged in the excretion area from a user may exist from the side of the first sheet 110 that is supposed to face the user to the third sheet 130.

The pattern 200 is provided to sense excretion discharged to the excretion area, so it may be disposed within a section from the first sheet 110 to the third sheet 130 where excretion may exist.

The pattern 200 can be printed or coated on a side of the first sheet 110 that faces the second sheet 120. For example, the pattern 200 may include a conductive pattern printed or coated on a side of the first sheet 110 that is supposed to face a user. When the first sheet 110 includes a material that passes some of excretion, the pattern 200 may be formed on an opposite side to the side of the first sheet that is supposed to face a user.

The conductive ink 10 may include at least one (conductive substance such as a carbon-based substance, a polymer-based substance, a metal oxide, and PEDOT) of substances including a metal oxide such as carbon that conducts electricity and a conductive polymer, a solvent that thins down a conductive substance such as a carbon-based substance, a polymer-based substance, a metal oxide, and PEDOT, and water and acetyl corresponding to a dissolvent of a conductive polymer.

PEDOT is poly(3,4-ethylenedioxythiophene).

A polymer may be largely classified into a performable polymer and a functional polymer. The performable polymer is based on the property of a polymer and is a polymer characterized by mechanical properties or thermal properties. The functional polymer is a polymer that shows specific properties in accordance with functional groups existing in the polymer of shows functionality by blending a functional substance into a polymer.

The conductive polymer is mainly used for conductive forming materials and conductive films, and is used in various fields such as transportation of an IC and a transparent electrode. These fields may include a technology of blending a conductive agent with a polymer.

The conductive polymer may include polyacetylene, polypyrrole, polythiophene, polyaniline, etc.

The first sheet 110 may include a transparent material such as polyethylene so that the pattern 200 printed or coated on a side of the first sheet 110 can be seen through the other side of the first sheet 110. As a result, as shown in FIG. 1, it is possible to easily find out whether the pattern 200 exists, and the position of the pattern 200, etc. through the other side of the first sheet 110.

Since the pattern 200 can be checked from the other side of the first sheet 110, a user can easily align the terminal unit 500 electrically connected to the pattern 200 to the sheet part 100.

A diaper may be formed larger than an actual excretion area to be worn on the body of a user. The absorber 150 may be disposed only in the excretion area k to improve productivity and fit.

Two or more patterns 200 may be provided, in which some patterns of a plurality of patterns 200 may form a positive (+) pattern 210 to which a sensing signal of the terminal unit 500 is applied, and the other patterns of the plurality of patterns 200 may form a negative (-) pattern 220 through which the sensing signal is sent back to the terminal unit 500.

The terminal unit 500 may be detachably attached to the sheet part 100. Three or more terminals 530 that are physically in contact with the patterns 200 may be formed at the terminal unit 500. In detail, the number of the terminals 530 may be larger by one or more than the number of the patterns 200.

The terminals 530 and the patterns 200 may be formed such that at least one terminal physically comes in contact with each of two or more patterns 200 spaced apart from each other when the terminal unit 500 is mounted on the sheet part 100.

For example, when two patterns 200 spaced a setting gap apart from each other are provided on the sheet part 100, two terminals 530 spaced the same gap as that of the two patterns 200 apart from each other may be provided at the terminal unit 500. In this case, when any one terminal 530 physically comes in contact with a specific pattern 200, the other terminal 530 can be naturally aligned to and physically brought in contact with the other pattern 200.

The terminals and the patterns 200 can be electrically connected by the physical contact between the terminals 530 and the patterns 200.

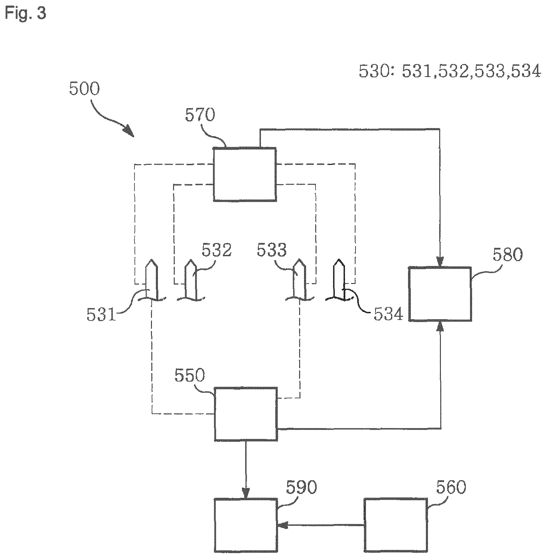

FIG. 3 is a block diagram showing the terminal unit 500.

The terminal unit 500 that is attached/detached to/from the sheet part 100 may include a sensing module 550, a determining module 570, a display module 580, a communication module 590, a counting module 560, etc.

The sensing module 550 can generate a sensing signal sensing excretion and provide the sensing signal to the terminal being in contact with the pattern 200.

For example, the sensing module 550 can apply a sensing signal to the terminal being in contact with the positive pattern 210 and can receive a sensing signal sequentially traveling through the positive pattern 210, excretion, and the negative pattern 220 from the terminal being in contact with the negative pattern 220. The sensing module 550 can sense whether there is excretion and the amount of excretion using the sensing signal input from the terminal being in contact with the negative pattern 220.

In order to sense a very small amount of excretion existing between the patterns 200 and improve the performance of sensing excretion, a first wing 211 protruding toward the negative pattern 220 may be provided at the positive pattern 210. Similar to the first wing 211, a second wing 221 protruding toward the positive pattern 210 may be provided at the negative pattern 220.

The terminal unit 500 may have a first attachment/detachment portion 510 and a second attachment/detachment portion 520 that are coupled to each other with the sheet part 100 therebetween in order to be attached/detached to/from the sheet part 100.

In this case, a terminal may be installed at the first attachment/detachment portion 510 disposed on a side of the first sheet 110 that is supposed to face a user. The terminal can physically come in contact with the pattern through the second sheet 120 when the terminal unit 500 is mounted on the sheet part 100.

Since the terminal is disposed on the side of the first sheet 110 that is supposed to face a user, it is possible to prevent a phenomenon in which an undesired hole is formed at the first sheet 110 by a terminal. As a result, according to the embodiment, the first sheet 110 can be maintained in a state in which excretion cannot pass through the first sheet 110.

The terminal unit 500 can find out whether there is a problem with the sheet part 100 or whether there is physical contact/alignment between the patterns 200 and the terminals using a plurality of terminals of which the number is larger than the number of the patterns 200.

For example, the terminal unit 500 may include a first terminal 531 and a second terminal 532 that are both in contact with one specific pattern 200 of a plurality of patterns 200.

The terminal unit 500 may include the determining module 570 that finds out the physical characteristic of one specific pattern 200 using the first terminal 531 and the second terminal 532 both being in contact with the specific pattern 200.

The determining module 570 can determine whether there is a problem with the sheet part 100 using the physical characteristic of the specific pattern 200.

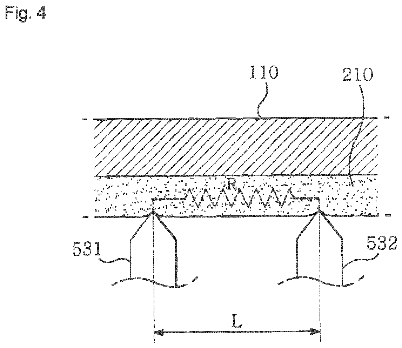

FIG. 4 is a schematic view showing the first terminal 531 and the second terminal 532 being in contact with a specific pattern 200.

For example, the terminal unit 500 may include a first terminal 531 and a second terminal 532 that are in contact with one specific pattern 200 (regardless of the negative pattern 220 and the positive pattern 210) of a plurality of patterns 200.

The terminal unit 500, in detail, the determining module 570 can measure a resistance value between the first terminal 531 and the second terminal 532, physically in contact with the specific pattern 200. In this case, the resistance value R measured by the determining module 570 may be a resistance value in the section of the pattern 200 disposed between the first terminal 531 and the second terminal 532.

When the distance between the first terminal 531 and the second terminal 532 is L, the resistance value R measured by the determining module 570 may be a resistance value per L.

The determining module 570 can compare a lookup table including the resistance value per the distance L and the measured resistance value R. When the measured resistance value R satisfies a setting value, as the result of comparison, the determining module 570 can determine that the corresponding pattern 200 and the corresponding sheet part 100 are normal.

When the measured resistance value R comes out of the setting value, the determining module 570 of the terminal unit 500 can generate a warning signal saying a problem with the sheet part 100 instead of a sensing signal. When the measured resistance value R comes out of the setting value, excretion may not be normally sensed or an electric shock accident may be caused by overcurrent, etc. According to the embodiment, when a problem with the pattern 200 is generated, a warning signal is generated instead of a sensing signal that may cause an electric shock, etc., so an electric shock accident can be prevented.

The warning signal generated by the determining module 570 can be output through the display module 580 of FIG. 3 such as a display, a warning light, and a speaker.

According to the embodiment, it is possible to not only easily find out whether there is a problem with the sheet part 100, but also recognize an imitative sheet not considering the safety of a user, etc.

Meanwhile, the determining module 570 can determine the alignment state between the terminals and the patterns 200 or whether there is physical contact between the terminals and the patterns 200.

For example, the first terminal 531 can provide a sensing signal to a specific pattern 200 of a plurality of patterns 200. In this case, the second terminal 531 may be formed to be in contact with the specific pattern 200 together with the first terminal 531.

The determining module 570 of the terminal unit 500 can determine an alignment state between the first terminal 531 and the specific pattern 200 or whether there is physical contact between the first terminal 531 and the specific pattern 200.

The first terminal 531 and the second terminal 532 connected to the same specific pattern 200 that conducts electricity may be electrically connected by the specific pattern 200.

In this case, the determining module 570 can apply a determination signal to one of the first terminal 531 and the second terminal 532 and can determine whether the first terminal 531 and the second terminal 532 are electrically connected by analyzing the determination signal sent back to the other one. The determination signal may be for testing the electrical conduction state between the first terminal 531 and the second terminal 532.

When determining that the first terminal 531 and the second terminal 532 are electrically connected, the determining module 570 can determine that both of the first terminal 531 and the second terminal 532 are normally in contact with the specific pattern 200. The fact that the first terminal 531 and the second terminal 532 are both in contact with the same specific pattern 200 may mean that the first terminal 531 and the second terminal 532 are aligned to the specific pattern 200.

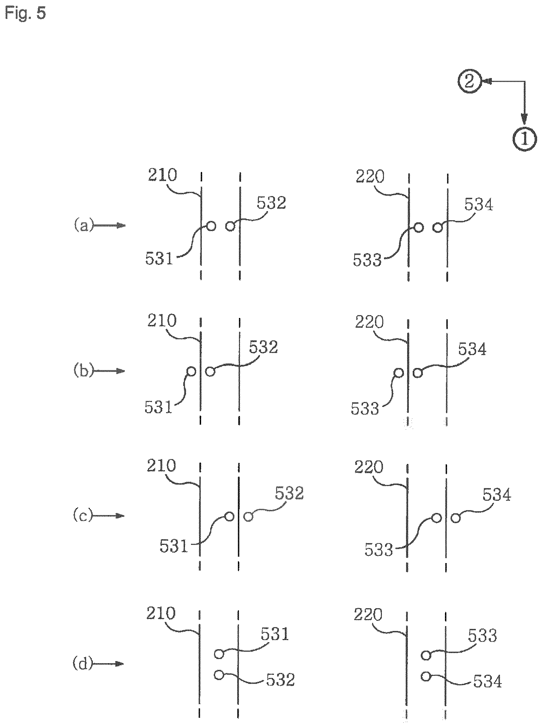

FIG. 5 illustrates schematic views showing an alignment state between patterns 200 and terminals. FIGS. 5(a), 5(b), 5(c), and 5(d) show an alignment state of terminals with patterns 200.

When the patterns 200 extend in a first direction ①, the patterns 200 may be formed to be spaced apart from each other in a second direction ② perpendicular to the first direction ①. In this case, the first direction ① may be the longitudinal direction of the sheet part 100 and the second direction ② may be the width direction of the sheet part 100.

In order to determine whether there is a problem with the patterns 200, whether the terminals and the patterns 200 are in contact with each other, and the alignment state between the terminals and the patterns 200, at least two terminals of three or more terminals provide at the terminal unit 500, for example, the first terminal 531 and the second terminal 532 both can be connected to any one specific pattern 200 of the plurality of patterns 200.

When a total of four terminals of a first terminal 531, a second terminal 532, a third terminal 533, and a fourth terminal 534 are provided at the terminal unit 500, the first terminal 531 and the second terminal 532 may be formed both to be in contact with the positive pattern 210. The third terminal 533 and the fourth terminal 534 may be formed both to be in contact with the negative pattern 220. The gap between the first terminal 531 and the third terminal 533 and the gap between the second terminal 532 and the fourth terminal 534 may be the same as the gap between the positive pattern 210 and the negative pattern 220 such that when the first terminal 531 and the second terminal 532 are aligned to the positive pattern 210, the third terminal 533 and the fourth terminal 534 are aligned to the negative pattern 220.

In this case, the first terminal 531 or the second terminal 532 may be an element that applies a sensing signal to the patterns 200. When excretion exists across the positive pattern 210 and the negative pattern 220, the excretion can function as a conductor that electrically connects the positive pattern 210 and the negative pattern 220. The third terminal 533 or the fourth terminal 534 can provide a sensing signal transmitted to the negative pattern 22 through excretion to the terminal unit 500.

The sensing module 550 can find out whether there is excretion or the amount of excretion by analyzing the sensing signal provided from the third terminal 533 or the fourth terminal 534.

When excretion is sensed, the sensing module 550 can display the fact that the excretion exists or the amount of the excretion through the display module 580.

For example, the sensing module 550 can control the display module 580 such that different colors are displayed in accordance with the amount of sensed excretion.

A plurality of terminals being in contact with one pattern 200, for example, the first terminal 531 and the second terminal 532 may be formed to be spaced apart from each other in the second direction ②, as shown in FIG. 5.

In order that the first terminal 531 and the second terminal 532 can be in contact with one same specific pattern 200, the first terminal 531 and the second terminal 532 may be formed with a gap under the width of the specific pattern 200 in the second direction ②. According to the embodiment, as shown in FIG. 5(a), the first terminal 531 and the second terminal 532 both may be in contact with the positive pattern 210 and the third terminal 533 and the fourth terminal 534 both may be in contact with the negative pattern 220.

Meanwhile, as shown in FIGS. 5(b) and 5(c), only one of the first terminal 531 and the second terminal 532 may be in contact with the positive pattern 210 and only one of the third terminal 533 and the fourth terminal 534 may be in contact with the negative pattern 220.

A determination signal provided to the first terminal 531 by the terminal unit 500 can be applied to a specific pattern 200 through the first pattern 531. The determination signal can be received to the second terminal 532 through the specific pattern 200. The determining module can additionally provide a determination signal through the third terminal 533. The determination signal provided to the third terminal can be received to the fourth terminal through the negative pattern

The determining module 570 of the terminal unit 500 can determine the physical characteristic of the specific pattern 200 or can determine whether the first terminal 531 and the specific pattern 200 are physically in contact with each other, using the determination signal received to the second terminal 532. The terminal unit 500 can determine whether to generate a sensing signal, depending on the result of determining the physical characteristic or the result of determining whether they are physically in contact with each other.

In the cases of FIGS. 5(b) and 5(c), one terminal is connected to each terminal 200, so normal operation of a sensing signal is possible. However, the determining module 570 can determine that the alignment state or contact state between the terminals and the patterns 200 is poor. A sensing signal may not be generated due to the determination of badness by the determining module 570.

Accordingly, in the cases of FIGS. 5(b) or 5(c), a user has to make the state shown in FIG. 5(a) by changing the mounted state of the terminal unit 500 on the sheet part 100. Therefore, the user has to endure inconvenience of precisely aligning the terminal unit 500 and the sheet part 100 to make the state shown in FIG. 5(a).

In order to reduce the inconvenience of a user, the first terminal 531 and the second terminal 532 may be disposed to be spaced apart from each other in the first direction ① and may be disposed at the same position in the second direction ②, as shown in FIG. 5(d).

According to the embodiment, even if the alignment state between the terminal unit 500 and the sheet part 100 slight breaks in the second direction ②, the first terminal 531 and the second terminal 532 both may be in contact with the positive pattern 210 and the third terminal 533 and the fourth terminal 534 both may be in contact with the negative pattern 220. As a result, according to the embodiment shown in FIG. 5(d), the precision required for the alignment between the terminal unit 500 and the sheet unit 100 can be attenuated.

Returning to FIG. 3, the communication module 590 can perform wireless communication with a server. For example, the communication module 590 can communicate with a server through a wireless communication network such as Zigbee, WiFi, and Bluetooth. It is preferable that the communication module 590 uses a Zigbee network in consideration of the communication distance.

When excretion is sensed by the sensing module 550, the communication module 590 can create and transmit a first notification message including recognition information of the terminal unit 500 and first visual information with the excretion sensed to the server.

The server receiving the first notification message can recognize a user wearing the terminal unit 500 using the recognition information of the terminal unit 500. The server can manage the excretion state of the recognized user using the first visual information about the sensed excretion.

On the other hand, the terminal unit 500 may include the counting module 560 that counts the number of sheet parts 100 every time the sheet parts 100 are replaced.

The communication module 590 can transmit a second notification message including recognition information of the terminal unit 500, information about the number of sheet parts 100 replaced for the terminal unit 500, and second visual information with the sheet parts 100 replaced to the server.

The information about the number of the replaced sheet parts 100 may be used to establish policies such as determining the amount to allowance such as medical insurance. The second visual information may be sued to find out the replacement habit of the sheet parts 100 by the user. In some cases, the second visual information may be used to expose an act of illegally replacing the sheet parts 100 for an abnormal demand for medical insurance.

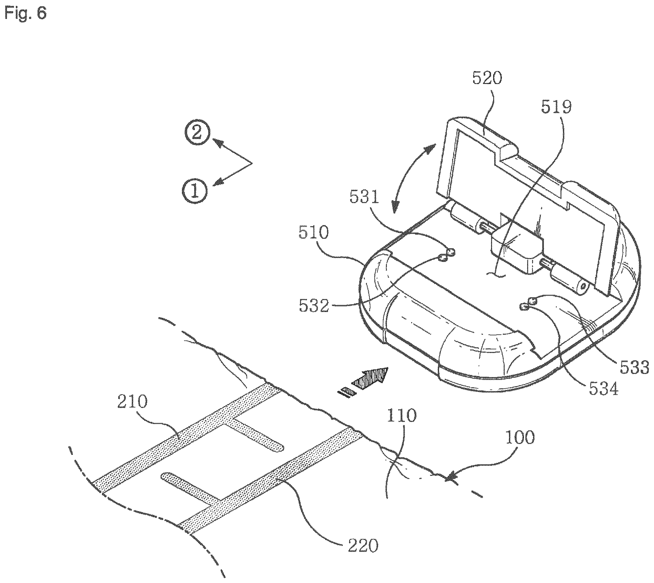

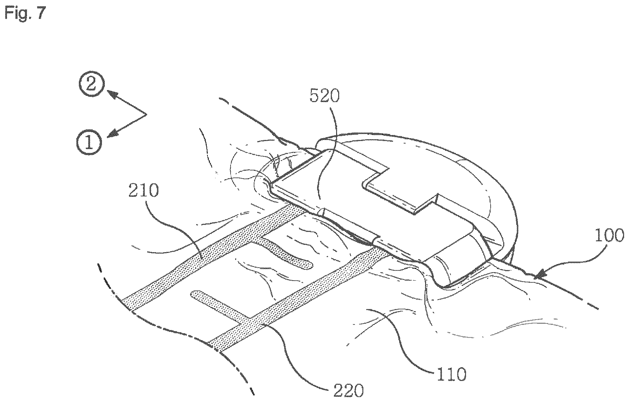

FIGS. 6 and 7 are schematic views showing the terminal unit 500 of the present invention.

A groove-shaped seat 519 in which the edge of a sheet may be formed at the first attachment/detachment portion 510 of the terminal unit 500 disposed at a position facing patterns 200. In this case, terminals may protrude from the seat 519.

A second attachment/detachment portion 520 of the terminal unit 500 can be formed rotatably with respect to the first attachment/detachment portion 510.

When the end of the sheet part 100 is put on the seat 519 such that the terminals and the patterns 200 are aligned with each other, and then the second attachment/detachment portion 520 is rotated to cover the seat 519, the of the sheet part 100 is fitted between the first attachment/detachment portion 510 and the second attachment/detachment portion 520, as in FIG. 7.

The patterns 200 may extend from a rear end to the other rear end of the sheet part 100 such that the patterns 200 are connected to the terminals of the terminal unit 500 mounted at an end of the sheet part 100. According to the embodiment, the terminal unit 500 may be mounted at an end or the other end of the sheet part 100.

In FIG. 6, the first terminal 531 and the second terminal 532 are disposed at the same position in the first direction ① and disposed to be spaced apart from each other in the second direction ②. In this case, as in FIG. 5(d), the first terminal 531 and the second terminal 532 may be disposed at different positions in the first direction ① and may be disposed at the same position in the second direction ②.

The patterns 200 may include a first pattern and a second pattern electrically separated from each other. For example, the first pattern may include the positive pattern 210. The second pattern may include the negative pattern 220.

The terminal unit 500 may include a first terminal 531 and a second terminal 532 formed to be able to come in contact with the first pattern and a third terminal 533 and a fourth terminal 534 formed to be able to come in contact with the second pattern.

When the first terminal and the second terminal both come in contact with the first pattern, the first terminal and the second terminal can be electrically connected by the first pattern.

When the third terminal and the fourth terminal both come in contact with the second pattern, the third terminal and the fourth terminal can be electrically connected by the second pattern.

The determining module can apply a first determination signal to any one of the first terminal and the second terminal and can apply a second determination signal to any one of the third terminal and the fourth terminal.

The determining module can check whether the first determination signal is received from the other one of the first terminal and the second terminal.

The determining module can check whether the second determination signal is received from the other one of the third terminal and the fourth terminal.

The sensing module can generate a sensing signal that can sense excretion only when both of the first determination signal and the second determination signal are received. According to the embodiment, a sensing signal can be generated only when four terminals are all aligned with patterns in contact with the patterns. Accordingly, a phenomenon in which a sensing signal is unnecessarily generated can be prevented.

The sensing module can apply the sensing signal to at least one of the first terminal and the second terminal. The sensing module can monitor a sensing signal that is received from at least one of the third terminal and the fourth terminal.

The sensing module can find out whether there is excretion or the amount of excretion by monitoring the sensing signal.

When power is applied, the determining module can operate in a slip mode that applies a determination signal for determining whether a terminal is in contact with a pattern to a terminal.

When a terminal is determined as being in contact with a pattern by the determining module operating in the slip mode, the sensing module can operate in an on-mode that applies a sensing signal to the terminal.

In this case, the determination signal may have a power value lower than the sensing signal. The sensing module and the determination module can be operated by a battery having a limited charge capacity and disposed in the terminal unit. In order to minimize the operation period of the sensing module or the determining module, the determination signal and the sensing signal may be periodically generated and applied to a terminal in accordance with a setting period without being generated in real time.

The determination signal can determine whether a normal sheet part is mounted on the terminal unit. According to the determination signal, a sensing signal is not generated even if a user connects a terminal using fingers, an imitative sheet, and other physical reactions. Accordingly, it is forced to use a normal sheet part, so it is possible to induce a user to use a normal sheet part that satisfies setting references such as an absorption ratio. Further, it is difficult to forge the amount of replaced sheet parts in order to abnormally receive an insurance amount.

In order to determine a sheet part that is not known about when to be mounted, a determination signal can be generated when a battery is newly mounted in the terminal unit or a power switch is turned on. As a result, a determination signal can be generated as long as power is applied to the terminal. In this case, the power value of the determination signal is lower than a sensing signal, unnecessary power consumption can be minimized.

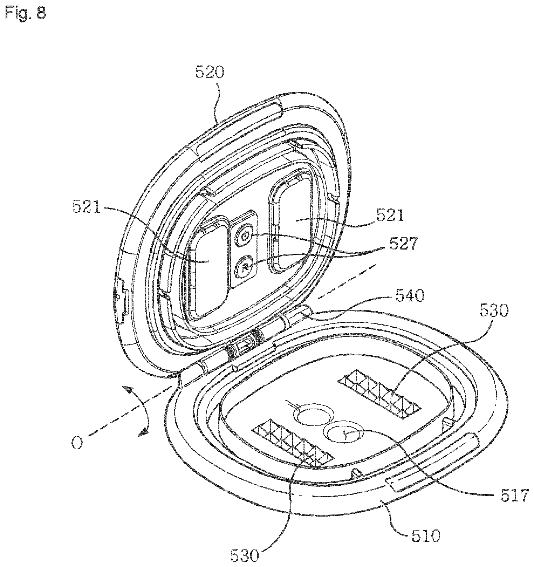

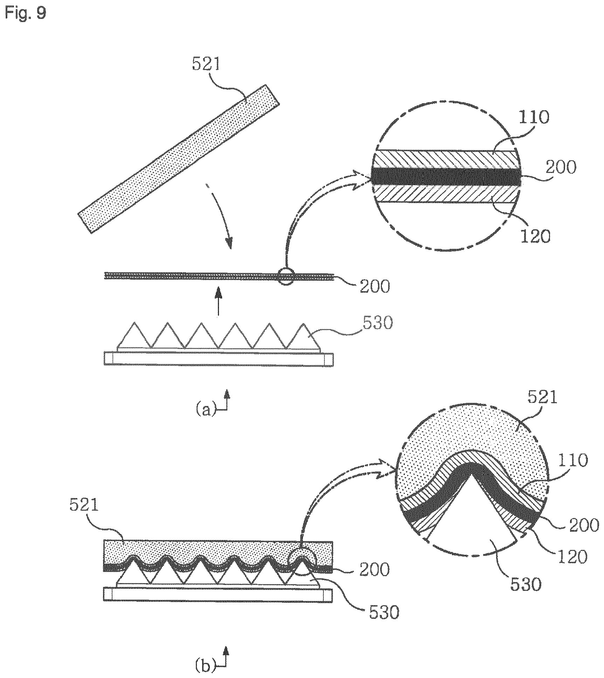

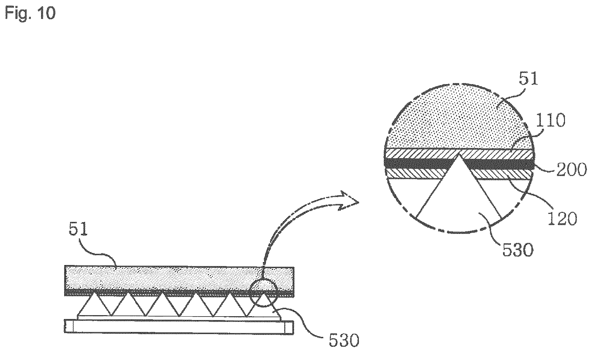

FIG. 8 is a schematic view showing another terminal unit of the present invention. FIG. 9 illustrates schematic views showing a state in which a sheet part is disposed between elastic parts and terminals. FIG. 10 is a schematic view showing a comparative embodiment of the present invention.

A terminal unit 500 may have a first attachment/detachment portion 510, a second attachment/detachment portion 520, and a hinge portion 540.

The first attachment/detachment portion 510 and the second attachment/detachment portion 520 can be attached/detached to/from the sheet part 100 by coming in contact with each other or moving away from each other with the sheet part 100 that holds excretion of a user therebetween.

Terminals 530 can come in contact with patterns 200 disposed on the sheet part 100 when the first attachment/detachment portion 510 and the second attachment/detachment portion 520 are mounted on the sheet part 100.

The hinge portion 540 can rotatably connect the rear end of the first attachment/detachment portion 510 and the rear end of the second attachment/detachment portion 520. The hinge portion 540 may be formed rotatably around a rotation axis O. The first attachment/detachment portion 510 and the second attachment/detachment portion 520 can come in contact with each other or move away from each other by rotating about the hinge portion 540.

The terminal 530 may be installed on the first attachment/detachment portion 510 of the first attachment/detachment portion 510 and the second attachment/detachment portion 520 coupled to each other with the sheet part 100 therebetween. In this case, the first attachment/detachment portion 510 can face the patterns.

Elastic parts 521 facing the terminals 530 with the sheet part 100 therebetween may be disposed on the second attachment/detachment portion 520. The elastic parts 521 may include an elastic member formed in a plate shape such as rubber.

The terminals 530 may protrude toward the elastic parts 521 to come in close contact with the elastic parts 521 with the first attachment/detachment portion 510 and the second attachment/detachment portion 520 in close contact with each other. When the first attachment/detachment portion 510 and the second attachment/detachment portion 520 are combined with each other, the terminals 530 come in contact with the patterns 200 disposed in the sheet part through the surface of the sheet part 100 supported on the elastic parts 521, thereby being able to press the elastic parts 521.

The terminals 530 may be formed in a cone shape with a pointed end to penetrate into the first sheet 110 or the second sheet 120. The first attachment/detachment portion may be disposed close to the first sheet and the second attachment/detachment portion may be disposed close to the second sheet. On the contrary, the first attachment/detachment portion may be disposed close to the second sheet and the second attachment/detachment portion may be disposed close to the first sheet.

When the first attachment/detachment portion and the second attachment/detachment portion are combined with each other, the terminals can press the elastic parts in contact with the patterns.

The elastic parts 521, as shown in FIG. 9, are recessed together with the patterns 200 by pressing of the terminals 530, whereby it is possible to bring the patterns 200 and the terminals 300 in close contact with each other using the recessing elastic reaction force. In this case, the patterns and the terminals can be in surface contact.

In the comparative example of FIG. 10 in which rigid members 51 are provided instead of the elastic parts 521, since the elastic parts 521 that can partially absorb pressing force of the terminals are removed, the terminals can penetrate into not only the second sheet part 120, but also the patterns 200. As a result, the terminals are fitted in holes formed in the patterns 200 and sides of the terminals may be spaced apart from the holes of the patterns 200 and electrically disconnected by fine movement. Even if the sides of the terminals are in contact with the holes of the patterns 200, it is linear contact, so the electrical connection state easily becomes poor. Further, the electrical property of the patterns 200 in which the holes are formed by the terminals is easily deteriorated.

According to the elastic parts 521 of the present invention, holes that are formed in the patterns 200 by the terminals 530 are securely prevented and the terminals and the patterns 200 are in surface contact, so the electrical connection state or the electrical property can be improved.

The terminal unit may have an input unit 527 that controls the sensing module and the determining module. The input unit 527 may include a power button for turning on or off the sensing module or the determining module, a reset button for resetting the sensing module or the determining module, etc.

It is required to prevent unexpected operation of the input unit 527 due to contact with the body of a user or the clothes that a user wears.

The input unit 527 may be installed on a side of the first attachment/detachment portion 510 that faces the second attachment/detachment portion 520 or a side of the second attachment/detachment portion 520 that faces the first attachment/detachment portion 510. In the first attachment/detachment portion 510, an accommodation groove 517 in which the input unit 527 protruding toward the first attachment/detachment portion 510 may be formed on the side facing the input unit 527.

Due to the characteristic of the terminals that have to penetrate into the first sheet or the second sheet and the characteristic of the sensing module that has to be spaced from excretion, it is preferable that the terminals and the sensing modules are separately installed at different attachment/detachment portions. Instead, wires electrically connecting the terminals and the sensing module may be provided between the first attachment/detachment portion 510 and the second attachment/detachment portion 520.

## Claims

1. An absorption product comprising:
a sheet part having a pattern that conducts electricity; and
a terminal unit having a terminal formed to be physically in contact with the patterns and being attached and detached to and from the sheet part,
wherein the pattern is formed as two or more pieces to be spaced apart from each other in an excretion area that receives excretion of a user,
a sensing module generating a sensing signal sensing the excretion is provided in the terminal unit, and
the sensing module provides the sensing module to the terminal being in contact with the pattern.

2. The absorption product of claim 1, wherein when the terminal unit is mounted on the sheet part, at least one terminal physically comes in contact with each of the two or more patterns spaced apart from each other,
the sensing module applies the sensing signal to a terminal being in contact with a positive (+) pattern,
the sensing module receives the sensing signal, which has sequentially passed through the positive pattern, the excretion, and a negative (-) pattern, from a terminal being in contact with the negative pattern,
a first terminal and a second terminal both being in contact with one specific pattern of a plurality of patterns are provided in the terminal unit, and
a determining module applying a determination signal to the first terminal and receiving the determination signal through the second terminal is provided.

3. The absorption product of claim 1, wherein a first terminal and a second terminal both being in contact with one specific pattern of a plurality of patterns are provided in the terminal unit,
a determining module finding out a physical characteristic of the one specific pattern using the first terminal and the second terminal both being in contact with the specific pattern is provided in the terminal, and
the determining module determines whether there is a problem with the sheet part using the physical characteristic of the specific pattern.

4. The absorption product of claim 1, wherein a first terminal and a second terminal both being in contact with one specific pattern of a plurality of patterns are provided in the terminal unit,
the terminal unit measures a resistance value between the first terminal and the second terminal physically in contact with the specific pattern, and
the terminal unit generates a warning signal saying a problem with the sheet instead of the sensing signal when the resistance value comes out of a setting value.

5. The absorption product of claim 1, wherein a fist terminal providing the sensing signal to a specific pattern of a plurality of patterns and a second terminal being in contact with the specific pattern together with the first terminal are provided in the terminal unit, and
the terminal unit determines an alignment state between the first terminal and the specific pattern or whether the first terminal and the specific pattern are physically in contact with each other, using the second terminal.

6. The absorption product of claim 1, wherein the pattern includes a first pattern and a second pattern that are electrically separated from each other,
a first terminal and a second terminal that are formed to be able to come in contact with the first pattern, and a third terminal and a fourth terminal that are formed to be able to come in contact with the second pattern are provided in the terminal unit,
when the first terminal and the second terminal both come in contact with the first pattern, the first terminal and the second terminal are electrically connected to each other by the first pattern,
when the third terminal and the fourth terminal both come in contact with the second pattern, the third terminal and the fourth terminal are electrically connected to each other by the second pattern,
the terminal unit applies a first determination signal to any one of the first terminal and the second terminal and applies a second determination signal to any one of the third terminal and the fourth terminal,
the terminal unit checks whether the first determination signal is received to the other one of the first terminal and the second terminal,
the terminal unit checks whether the second determination signal is received to the other one of the third terminal and the fourth terminal,
the sensing module generates the sensing signal only when the first determination signal and the second determination signal are both received,
the sensing module applies the sensing signal to at least one of the first terminal and the second terminal and monitors the sensing signal that is received from at least one of the third terminal and the fourth terminal, and
the sensing module finds out whether there is the excretion or the amount of the excretion by monitoring the sensing signal.

7. The absorption product of claim 1, wherein the terminal unit operates in a slip mode that applies a determination signal determining whether the terminal is in contact with the pattern to the terminal when power is applied,
the terminal operates in an on-mode that applies the sensing signal to the terminal when the terminal is determined as being in contact with the pattern while operating in the slip mode, and
the determination signal has a power value lower than the sensing signal.

8. The absorption product of claim 1, wherein a communication module performing wireless communication with a server is further provided in the terminal unit, and
when the excretion is sensed by the sensing module, the communication module generates and transmits a first notification message including recognition information of the terminal unit and first visual information with the excretion sensed to the server.

9. The absorption product of claim 1, wherein a counting module counting the number of replaced sheet parts every time the sheet parts are replaced, and a communication module performing wireless communication with a server, and
the communication module transmits a second notification message including recognition information of the terminal unit, information about the number of the sheet parts replaced for the terminal unit, and second visual information with the sheet parts replaced to the server.

10. The absorption product of claim 1, wherein the sheet part includes a first sheet made of a material at least partially not passing the excretion and a second sheet stacked on a side of the first sheet, which is supposed to face the user, and passing the excretion,
the pattern is printed on a side of the first sheet that faces the second sheet,
a first attachment/detachment portion and a second attachment/detachment portion combined with each other with the sheet part therebetween are provided in the terminal unit,
the terminal is installed in the first attachment/detachment portion disposed on the side of the first sheet that is supposed to face the user, and
the terminal comes physically in contact with the pattern through the second sheet when the terminal unit is mounted on the sheet part.

11. The absorption product of claim 1, wherein a first attachment/detachment portion, a second attachment/detachment portion, and a hinge portion are provided in the terminal unit,
the first attachment/detachment portion and the second attachment/detachment portion are attached and detached to and from the sheet part by coming in contact with each other or moving away from each other with the sheet part therebetween,
the hinge portion rotatably connects the first attachment/detachment portion and the second attachment/detachment portion, and
the first attachment/detachment portion and the second attachment/detachment portion come in contact with each other or move away from each other by rotating about the hinge portion.

12. The absorption product of claim 1, wherein a first attachment/detachment portion and a second attachment/detachment portion combined with each other with the sheet part therebetween are provided in the terminal unit,
the terminal is installed in the first attachment/detachment portion facing the pattern,
an elastic part facing the terminal with the sheet part therebetween is provided in the second attachment/detachment portion, and
the terminal protrudes toward the elastic part.

13. The absorption product of claim 12, wherein when the attachment/detachment portion and the second attachment/detachment portion are combined, the terminal presses the elastic part in contact with the pattern, and
the elastic part is recessed together with the pattern by pressing of the terminal and brings the pattern and the terminal in close contact with each other using a recessing elastic reaction force.

14. The absorption product of claim 12, wherein the terminal is formed is a cone shape, and
when the first attachment/detachment portion and the second attachment/detachment portion are combined with each other, the terminal comes in contact with the pattern disposed in the sheet part through a surface of the sheet part supported on the elastic part and presses the elastic part.

15. The absorption product of claim 1, wherein a first attachment/detachment portion and a second attachment/detachment portion combined with each other with the sheet part therebetween are provided in the terminal unit,
the terminal is installed in the first attachment/detachment portion facing the pattern,
the sensing module is installed in the second attachment/detachment portion, and
a wire electrically connecting the terminal and the sensing module is provided between the first attachment/detachment portion and the second attachment/detachment portion.
